# EUROPEAN PATENT APPLICATION

(11) **EP 4 241 896 A1**
(43) Date of publication of application: **13.09.2023**
(21) Application number: 21888414.6
(22) Date of filing: 22.10.2021
(51) Int. Cl.: B08B 3/02, B08B 1/00, B08B 13/00, A61L 2/07

(54) **STEAM CLEANING DEVICE**

(30) Priority: 04.11.2020 CN 202011219389
(71) Applicant: Alfred Kärcher SE & Co. KG, 71364 Winnenden (DE)
(72) Inventor: LIU, Fei, Changshu, Jiangsu 215500 (CN); TANG, Xiaodong, Changshu, Jiangsu 215500 (CN); LIANG, Dandan, Changshu, Jiangsu 215500 (CN); AN, Yuxiang, Changshu, Jiangsu 215500 (CN)
(74) Representative: Hoeger, Stellrecht & Partner Patentanwälte mbB
(86) International application number: PCT/CN2021/125453
(87) International publication number: WO 2022/095717

(57) **Abstract**

A steam cleaning device (10), comprising: a box body (12) internally provided with a housing space (14), the housing space (14) comprising a plurality of mutually independent storage portions (16); a steam generating device (18) located inside the box body (12) and outside the housing space (14); and accessories (20) capable of being in fluid communication with the steam generating device (18) or being connected to each other, and suitable for being correspondingly housed in the plurality of mutually independent storage portions (16).

## Description

### CROSS-REFERENCE TO RELATED APPLICATIONS

This application claims the benefit of priority to Chinese patent application No. 202011219389.0, filed on November 4, 2020, entitled "STEAM CLEANING DEVICE", the entire disclosures of which are incorporated herein by reference.

### TECHNICAL FIELD

The present disclosure relates to a steam cleaning device.

### BACKGROUND

Steam cleaning devices are tools for dissolving stubborn dirt attached to objects and performing sterilization and disinfection by high-temperature steam. For example, the team cleaning devices can be used for sterilization, fine dust removal, decontamination, oil stain removal, and are widely used in personal and household deep cleaning and housekeeping fields.

However, the existing steam cleaning devices have a limited housing space, which cannot meet storage requirements of various accessories such as steam pipes and steam nozzles, and often cause damage to the accessories due to displacement caused by vibration when the accessories are transported for a long distance in the using process.

### SUMMARY

An object of the present disclosure is to provide an improved steam cleaning device.

An embodiment of the present disclosure provides a steam cleaning device. The steam cleaning device includes: a box body internally provided with a housing space, wherein the housing space includes a plurality of mutually independent storage portions; a steam generating device arranged inside the box body and outside the housing space; and an accessory capable of being in fluid communication with or connected with the steam generating device and adapted to be correspondingly accommodated in the plurality of mutually independent storage portions.

With this arrangement, it is beneficial for the accessory to be independently stored in the housing space of the steam cleaning device, and it is beneficial to prevent the accessory from being damaged due to vibration and displacement.

In some embodiments, the housing space is U-shaped.

With this arrangement, it is beneficial to obtain a high storage efficiency of the housing space and a compact structure of the steam cleaning device.

In some embodiments, the box body includes an upper cover, a lower cover arranged below the upper cover and a middle frame arranged between the upper cover and the lower cover, and the housing space is arranged in the middle frame.

With this arrangement, it is beneficial to obtain a more reasonable arrangement of various components and a more compact structure of the steam cleaning device.

In some embodiments, the housing space is arranged at an upper edge of the middle frame, the steam generating device is arranged below the middle frame, and the housing space partially surrounds the steam generating device.

With this arrangement, it is beneficial to arrange the steam generating device in a central area of the entire steam cleaning device and ensure that the center of gravity of the entire steam cleaning device is centered, which can reduce wrist pain caused by eccentric handling.

In some embodiments, the accessory includes a water tank that is in fluid communication with the steam generating device, and the plurality of mutually independent storage portions include a first storage portion adapted to accommodate the water tank.

With this arrangement, it is beneficial to use the steam cleaning device at any location and is not limited by a location of external water sources.

In some embodiments, the water tank includes a cover made of an elastomer.

With this arrangement, it is beneficial for the cover to absorb the vibration generated when the water tank moves.

In some embodiments, an inner side of the upper cover is provided with a support rib that abuts against an upper part of the cover.

With this arrangement, it is beneficial to provide a pre-tightening force for the cover of the water tank and fix the cover of the water tank, which can stabilize the water tank during transportation and reduce damage caused by displacement.

In some embodiments, the water tank includes a handle portion arranged outside a vertical line where a center of gravity of the water tank is located.

With this arrangement, it is beneficial to rotate the water tank to a stable position through its own gravity during carrying, and it is beneficial for a carrying position of the water tank to be more stable.

In some embodiments, in a horizontal direction, the handle portion is arranged between the center of gravity of the water tank and the cover.

With this arrangement, it is beneficial to keep the water in the water tank away from the cover of the water tank during carrying and reduce the risk of water leakage.

In some embodiments, the water tank is adapted to be removably accommodated in the first storage portion.

With this arrangement, it is beneficial for operations such as filling and cleaning the water tank.

In some embodiments, the water tank is adapted to be fixedly accommodated in the first storage portion.

With this arrangement, it is beneficial to stabilize the water tank.

In some embodiments, the housing space includes a second storage portion in addition to the first storage portion, and the second storage portion is L-shaped.

With this arrangement, it is beneficial for the center of gravity of the entire steam cleaning device to be centered, which can reduce wrist pain caused by eccentric handling.

In some embodiments, a bottom of the first storage portion is provided with a reference surface and an inlet, and a height of the inlet is greater than a height of the reference surface relative to an outer bottom surface of the first storage portion.

With this arrangement, it is beneficial to reduce the risk of pollutants particles clogging the inlet.

In some embodiments, an inner side of the upper cover is provided with a holding portion made of a soft rubber material, and the plurality of mutually independent storage portions include a third storage portion opposite to the holding portion.

With this arrangement, it is beneficial to reduce vibration during transportation.

In some embodiments, the accessory includes a steam nozzle and a steam pipe, the holding portion is adapted to accommodate the steam nozzle, and the third storage portion is adapted to accommodate the steam pipe.

With this arrangement, it is beneficial to reduce vibration and damage of the steam pipe by the holding portion absorbing vibration and contacting the steam pipe.

In some embodiments, a front outer side of the lower cover is provided with a connecting port for connecting the steam pipe.

With this arrangement, it is beneficial to disassemble the steam pipe and prevent damage to the steam pipe due to closing the upper cover during the use of the steam cleaning device.

In some embodiments, the accessory includes a power wire, a side bottom of the box body is provided with a wire outlet, and the power wire extends from the wire outlet to an outer side of the box body.

With this arrangement, it is beneficial to close the upper cover and use the steam cleaning device without damaging the power wire.

In some embodiments, at least one side of the box body is externally provided with a mesh portion for accommodating the power wire.

With this arrangement, it is beneficial for the storage and protection of the power wire.

In some embodiments, the steam cleaning device includes a handle arranged on an outer side of the upper cover.

With this arrangement, it is beneficial for the portability of the steam cleaning device.

In some embodiments, an end of the handle is provided with a mounting guide portion, and the outer side of the upper cover is provided with a mounting portion for matching with the mounting guide portion.

With this arrangement, it is beneficial to fix the handle on the upper cover conveniently and easily.

In some embodiments, the mounting guide portion has a mounting guide angle θ ranging from 30 to 40 degrees.

With this arrangement, it is beneficial to simplify the guide portion and fix the handle stably.

In some embodiments, the upper cover is capable of being opened and closed relative to the lower cover.

With this arrangement, it is beneficial to obtain easy operation.

In some embodiments, a front side of the upper cover is provided with a lock for locking the box body.

With this arrangement, it is beneficial to close the box body stably and ensure convenient and smooth transportation of the steam cleaning device.

In some embodiments, two sides of the lock are provided with a step portion respectively, and the upper cover includes a matching portion for matching with the step portion.

With this arrangement, it is beneficial to enhance a locking strength.

In some embodiments, a bottom of the box body is provided with a universal wheel.

With this arrangement, it is beneficial to move the steam cleaning device conveniently and easily.

The subject matter of any independent claim above may be combined with any single subject matter or combination of subject matter of any dependent claims to form new claimed subject matter where technical conditions permit.

The present disclosure will be further described with reference to the accompanying drawings. The same or similar reference numerals may be used in the drawings to refer to the same or similar elements in different embodiments, and the description of the same or similar elements in different embodiments may be omitted.

### BRIEF DESCRIPTION OF THE DRAWINGS

FIG. 1 is a schematic partial exploded perspective view of a steam cleaning device when an upper cover is opened according to an embodiment of the present disclosure;
FIG. 2 is another schematic exploded perspective view of the steam cleaning device in FIG. 1;
FIG. 3 is a schematic top view of the steam cleaning device in FIG. 1;
FIG. 4 is a schematic view of the connection between a steam generating device and a water tank, a steam pipe and a steam handle of the steam cleaning device in FIG. 1;
FIG. 5 is a schematic front view of a water tank of the steam cleaning device in FIG. 1;
FIG. 6 is a schematic top view of a middle frame and a lower cover of a box body of the steam cleaning device in FIG. 1;
FIG. 7 is a schematic partial sectional view of the box body in FIG. 6;
FIG. 8 is a schematic perspective view of the steam cleaning device in FIG. 1 when the upper cover is closed;
FIG. 9 is a schematic perspective view of a steam cleaning device when an upper cover is closed according to another embodiment of the present disclosure;
FIG. 10 is a schematic partial exploded perspective view of the upper cover of the steam cleaning device in FIG. 9;
FIG. 11 is another schematic partial exploded perspective view of the upper cover of the steam cleaning device in FIG. 9; and
FIG. 12 is a schematic partial enlarged view of the upper cover of the steam cleaning device shown in FIG. 11, in which a step portion and a matching portion are enlargedly shown.

### DETAILED DESCRIPTION

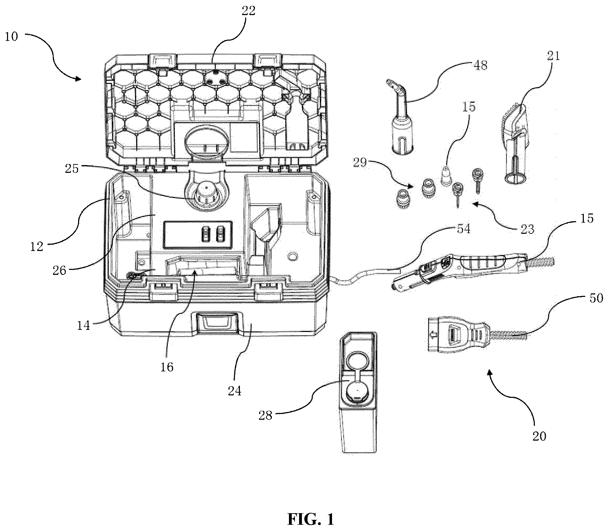

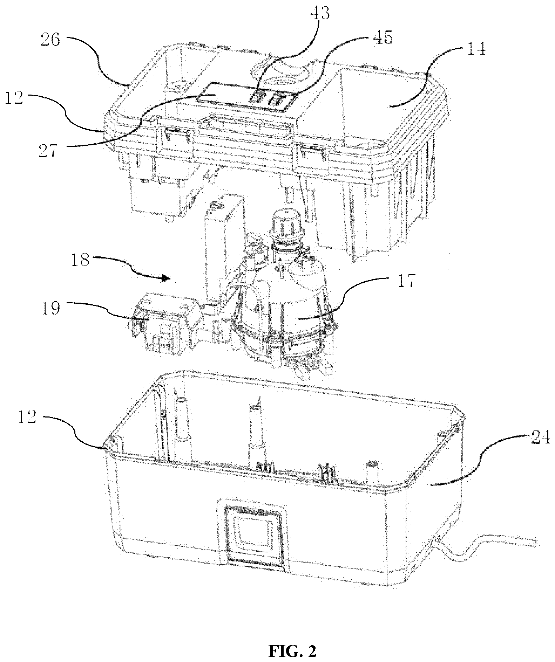

FIG. 1 is a schematic partial exploded perspective view of a steam cleaning device when an upper cover is opened according to an embodiment of the present disclosure. FIG. 2 is another schematic exploded perspective view of the steam cleaning device in FIG. 1. As shown in FIGS. 1 and 2, a steam cleaning device 10 includes a box body 12, a steam generating device 18 and an accessory 20. The box body 12 is internally provided with a housing space 14, and the housing space 14 includes a plurality of mutually independent storage portions 16. The steam generating device 18 is arranged inside the box body 12 and outside the housing space 14. The accessory 20 is capable of being in fluid communication with or connected with the steam generating device 18 and adapted to be correspondingly accommodated in the plurality of mutually independent storage portions 16.

With this arrangement, it is beneficial for the accessory 20 to be independently stored in the housing space 14 of the steam cleaning device 10, and it is beneficial to prevent the accessory 20 from being damaged due to vibration and displacement.

The steam generating device 18 may include a steam generator 17 (such as a boiler) and a water pump 19 that are in fluid communication with each other.

The accessory 20 includes, for example, a water tank 28, a steam pipe 50, a steam handle 13, a steam nozzle 48, a steam brush 21, a circular brush 29, a detail nozzle 15, a blade 23, and the like.

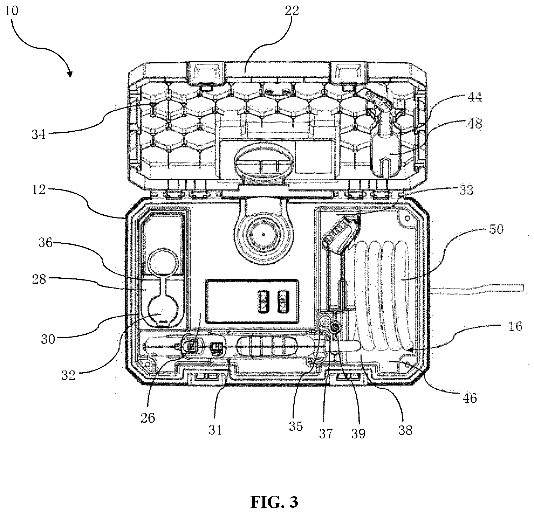

FIG. 3 is a schematic top view of the steam cleaning device in FIG. 1. As shown in FIGS. 1 and 3, the plurality of mutually independent storage portions 16 include a first storage portion 30, a second storage portion 38, a third storage portion 46, a fourth storage portion 31, a fifth storage portion 33, a sixth storage portion 35, a seventh storage portion 37, and an eighth storage portion 39 separated from each other.

The first storage portion 30 is adapted to accommodate the water tank 28. The second storage portion 38 is a portion of the housing space 14 in addition to the first storage portion 30. The third storage portion 46 is adapted to accommodate the steam pipe 50. The fourth storage portion 31 is adapted to accommodate the steam handle 13. The fifth storage portion 33 is adapted to accommodate the steam brush 21. The sixth storage portion 35 is adapted to accommodate the circular brush 29. The seventh storage portion 37 is adapted to accommodate the detail nozzle 15. The eighth storage portion 39 is adapted to accommodate the blade 23. The steam nozzle 48 is adapted to be accommodated in the holding portion 44.

In some embodiments, the housing space 14 is U-shaped.

With this arrangement, it is beneficial to obtain a high storage efficiency of the housing space 14 and a compact structure of the steam cleaning device 10.

The housing space 14 may have any suitable configuration as long as it is suitable for the present disclosure.

As shown in FIGS. 1 and 2, the box body 12 includes an upper cover 22, a lower cover 24 arranged below the upper cover 22 and a middle frame 26 arranged between the upper cover 22 and the lower cover 24, and the housing space 14 is arranged in the middle frame 26.

With this arrangement, it is beneficial to obtain a more reasonable arrangement of various components and a more compact structure of the steam cleaning device 10.

The middle frame 26 may be provided with components such as an adjustment knob 25 and an indication panel 27. The adjustment knob 25 may be used to adjust an amount of water, etc. The display panel 27 may be equipped with an indicator light 43 and a power switch 45, which may be used to indicate current operating status of the steam cleaning device 10, thus making operation more convenient and controllable.

The box body 12 may have any suitable configuration, and the housing space 14 may be arranged at other suitable locations as long as it is suitable for the present disclosure.

In some embodiments, the housing space 14 is arranged at an upper edge of the middle frame 26, the steam generating device 18 is arranged below the middle frame 26, and the housing space 14 partially surrounds the steam generating device 18.

With this arrangement, it is beneficial to arrange the steam generating device 18 in a central area of the entire steam cleaning device 10 and ensure that the center of gravity of the entire steam cleaning device 10 is centered, which can reduce wrist pain caused by eccentric handling.

Those skilled in the art can understand that the steam generating device 18 is a heavy component in the entire steam cleaning device 10. When the steam generating device 18 is arranged in the central area of the steam cleaning device 10, the center of gravity of the entire steam cleaning device 10 is centered, which can reduce wrist pain caused by eccentric handling.

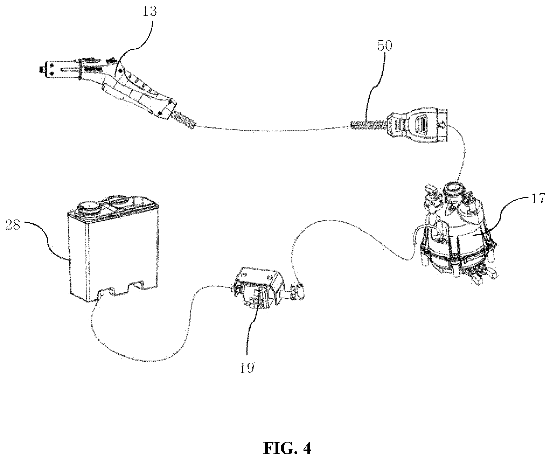

FIG. 4 is a schematic view of the connection between the steam generating device and the water tank, the steam pipe and the steam handle of the steam cleaning device in FIG. 1. As shown in FIGS. 1, 3 and 4, in some embodiments, the accessory 20 includes a water tank 28 that is in fluid communication with the steam generating device 18, and the plurality of mutually independent storage portions 16 include a first storage portion 30 adapted to accommodate the water tank 28.

With this arrangement, it is beneficial to use the steam cleaning device 10 at any location and is not limited by a location of external water sources.

Referring to FIG. 4, specifically, an outlet of the water tank 28 is communicated with an inlet of the water pump 19 of the steam generating device 18 to provide a water source for the steam cleaning device 10. An outlet of the water pump 19 is communicated with an inlet-outlet opening of the boiler 17 to provide water for the boiler 17. The boiler 17 is communicated with the steam handle 13 through the steam pipe 50 and transmits generated steam to the steam handle 13.

The water tank 28 is accommodated in the first storage portion 30 in the plurality of mutually independent storage portions 16, which enables to use the steam cleaning device 10 at arbitrary positions without being limited by the location of external water sources or using additional buckets for water supply.

In some embodiments, the water tank 28 includes a cover 32 made of an elastomer.

With this arrangement, it is beneficial for the cover 32 to absorb the vibration generated when the water tank 28 moves.

The cover 32 may be arranged, for example, on a top side of the water tank 28 and may have any suitable shape, such as a substantially circular shape. In addition, the cover 32 may have a sealing function to prevent water leakage in the water tank 28. The cover 32 may be manufactured by any suitable elastomer as long as it has the effect of absorbing vibration. For example, the elastomer may include Thermoplastic Elastomer (TPE), silicone, Ethylene Propylene Diene Monomer (EPDM), and the like.

In some embodiments, an inner side of the upper cover 22 is provided with a support rib 34 that abuts against an upper part of the cover 32.

With this arrangement, it is beneficial to provide a pre-tightening force for the cover 32 of the water tank and fix the cover 32 of the water tank, which can stabilize the water tank 28 during transportation and reduce damage caused by displacement.

The support rib 34 may be arranged on the inner side of the upper cover 22 opposite to the cover 32 of the water tank and may have a shape corresponding to the cover 32. The support rib 34 may be made of, for example, polycarbonate acrylonitrile butadiene styrene (PC-ABS).

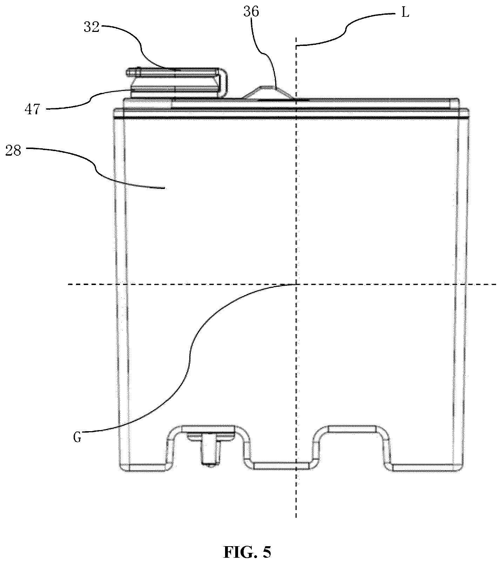

FIG. 5 is a schematic front view of the water tank of the steam cleaning device in FIG. 1. As shown in FIGS. 3 and 5, the water tank 28 includes a handle portion 36 arranged outside a vertical line where a center of gravity G of the water tank 28 is located.

With this arrangement, it is beneficial to rotate the water tank 28 to a stable position through its own gravity during carrying, and it is beneficial for a carrying position of the water tank 28 to be more stable.

The water tank 28 may have an approximately rectangular shape. The handle portion 36 may be arranged, for example, at a top of the water tank 28, for example, near the cover 32. The handle portion 36 may have a substantially convex structure extending along a partial top surface of the water tank 28 away from an inlet 47 of the water tank 28.

Referring to FIG. 5, in some embodiments, in a horizontal direction, the handle portion 36 is arranged between the center of gravity G of the water tank 28 and the cover 32.

With this arrangement, it is beneficial to keep the water in the water tank 28 away from the cover 32 of the water tank during carrying and reduce the risk of water leakage.

In some embodiments, the water tank 28 is adapted to be removably accommodated in the first storage portion 30.

With this arrangement, it is beneficial for operations such as filling and cleaning the water tank 28.

In some embodiments, the water tank 28 is adapted to be fixedly accommodated in the first storage portion 30.

With this arrangement, it is beneficial to stabilize the water tank 28.

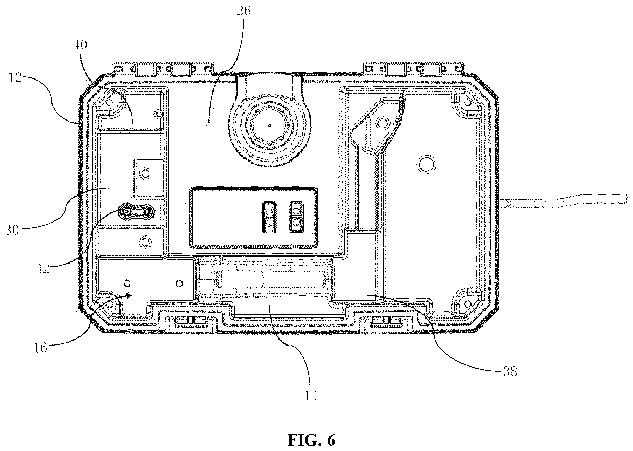

FIG. 6 is a schematic top view of the middle frame and the lower cover of the steam cleaning device in FIG. 1. As shown in FIG. 6, in some embodiments, the housing space 14 includes a second storage portion 38 in addition to the first storage portion 30, and the second storage portion 38 is L-shaped.

With this arrangement, it is beneficial for the center of gravity of the entire steam cleaning device 10 to be centered, which can reduce wrist pain caused by eccentric handling.

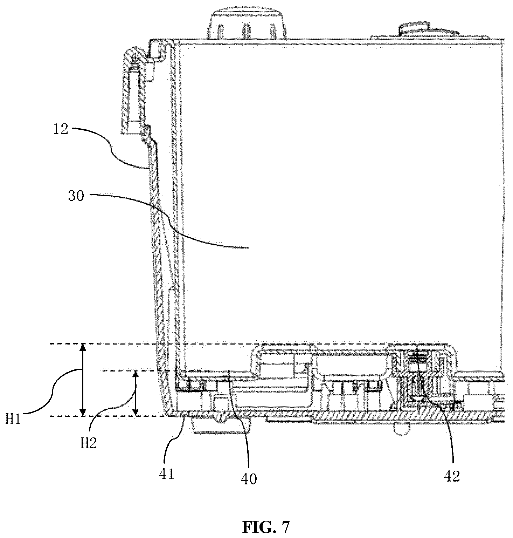

FIG. 7 is a partial sectional view of the box body in FIG. 6. As shown in FIGS. 6 and 7, a bottom of the first storage portion 30 is provided with a reference surface 40 and an inlet 42, and a height H1 of the inlet 42 is greater than a height H2 of the reference surface 40 relative to an outer bottom surface 41 of the first storage portion 30.

With this arrangement, it is beneficial to reduce the risk of pollutants particles clogging the inlet 42.

The water tank 28 may become contaminated with contaminants, posing a risk of clogging the inlet 42. It is found through water spraying tests that when the height H1 of the inlet 42 is greater than the height H2 of the reference surface 40, pollutant particles are not easily accumulated in the inlet 42, reducing the risk of clogging the inlet 42.

Still referring to FIG. 3, in some embodiments, an inner side of the upper cover 22 is provided with a holding portion 44 made of a soft rubber material, and the plurality of mutually independent storage portions 16 include a third storage portion 46 opposite to the holding portion 44.

With this arrangement, it is beneficial to reduce vibration during transportation.

The soft rubber material may be any suitable rubber material as long as it can absorb vibration.

Still referring to FIGS. 1 and 3, the accessory 20 includes a steam nozzle 48 and a steam pipe 50. The holding portion 44 is adapted to accommodate the steam nozzle 48, and the third storage portion 46 is adapted to accommodate the steam pipe 50.

With this arrangement, it is beneficial to reduce vibration and damage of the steam pipe 50 by the holding portion 44 absorbing vibration and contacting the steam pipe 50.

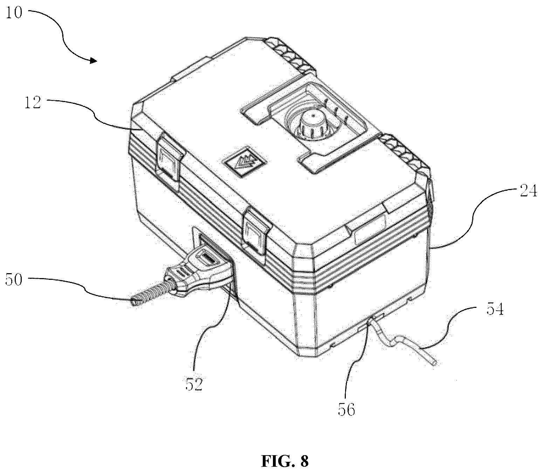

FIG. 8 is a schematic perspective view of the steam cleaning device in FIG. 1 when the upper cover is closed. In some embodiments, a connecting port 52 is provided on a front outer side of the lower cover 22, and the connecting port 52 is adapted to connect the steam pipe 50.

With this arrangement, it is beneficial to disassemble the steam pipe 50 and prevent damage to the steam pipe 50 due to closing the upper cover 22 during the use of the steam cleaning device 10.

Referring to FIGS. 1 and 8, the accessory 20 includes a power wire 54, and a side bottom of the box body 12 is provided with a wire outlet 56. The power wire 54 extends from the wire outlet 56 to an outer side of the box body 12.

With this arrangement, it is beneficial to close the upper cover 22 and use the steam cleaning device 10 without damaging the power wire 54.

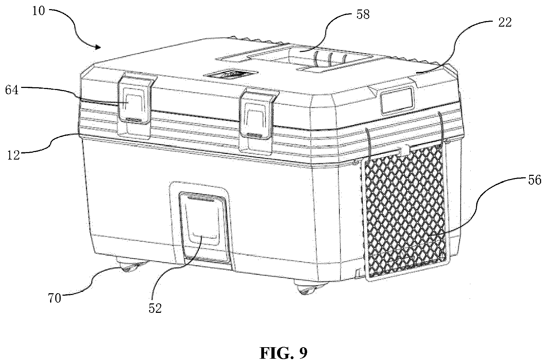

FIG. 9 is a schematic perspective view of a steam cleaning device when the upper cover is closed according to another embodiment of the present disclosure. As shown in FIG. 9, at least one side of the box body 12 is externally provided with a mesh portion 56 for accommodating the power wire 54.

With this arrangement, it is beneficial for the storage and protection of the power wire 54.

The power wire 54 is connected to the steam cleaning device 10 through the wire outlet 56 arranged outside the box body 12. The wire outlet 56 may be arranged inside the mesh portion 56. After use, the power wire 54 may be conveniently stored in the mesh portion 56, thereby obtaining additional protection from damage.

The mesh portion 56 may be directly assembled outside a side portion of the box body 12 through an assembly portion (not shown) arranged inside the side portion of the box body 12. Thus, the assembly process is simple and material costs for the steam cleaning device 10 can be saved.

In some embodiments, the steam cleaning device 10 includes a handle 58 arranged on an outer side of the upper cover 22.

With this arrangement, it is beneficial for the portability of the steam cleaning device 10.

The handle 58 may be approximately C-shaped and may be arranged, for example, in a center of the outer side of the upper cover 22.

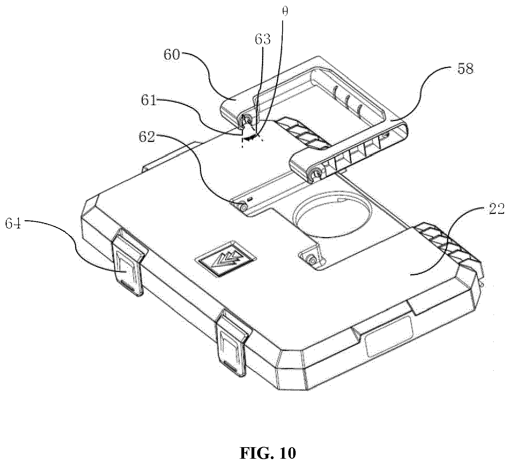

FIG. 10 is a schematic partial exploded perspective view of the upper cover of the steam cleaning device in FIG. 9. In some embodiments, an end of the handle 58 is provided with a mounting guide portion 60, and the outer side of the upper cover 22 is provided with a mounting portion 62 for matching with the mounting guide portion 60.

With this arrangement, it is beneficial to fix the handle 58 on the upper cover 22 conveniently and easily.

The mounting guide portion 60 may have a cylindrical shape with a notch on a side thereof. The mounting portion 62 may have a cylindrical shape and size corresponding to the mounting guide portion 60.

In some embodiments, the mounting guide portion 60 has a mounting guide angle θ ranging from 30 to 40 degrees.

In the present disclosure, the mounting guide angle θ refers to an angle of two corresponding contact points between two edges of the notch on the side surface of the substantially cylindrical mounting guide portion 60 and the substantially cylindrical mounting portion 62 relative to tangent lines 61 and 63 of the mounting portion 62 respectively.

Referring to FIGS. 1, 3 and 8-9, in some embodiments, the upper cover 22 can be opened and closed relative to the lower cover 24.

With this arrangement, it is beneficial to obtain easy operation.

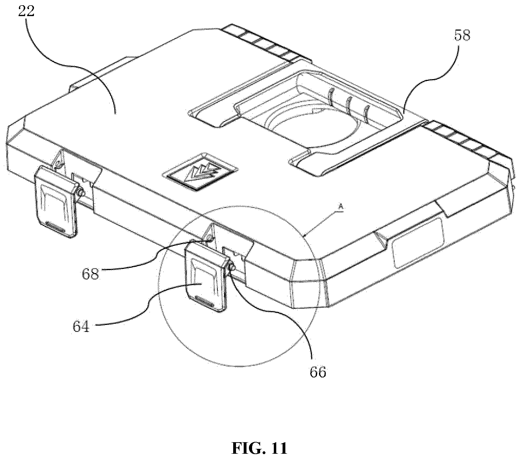

FIG. 11 is another schematic partial exploded perspective view of the upper cover of the steam cleaning device in FIG. 9. As shown in FIG. 11, in some embodiments, a front side of the upper cover 22 is provided with a lock 64 suitable for locking the box body 12.

With this arrangement, it is beneficial to close the box body 12 stably and ensure convenient and smooth transportation of the steam cleaning device 10.

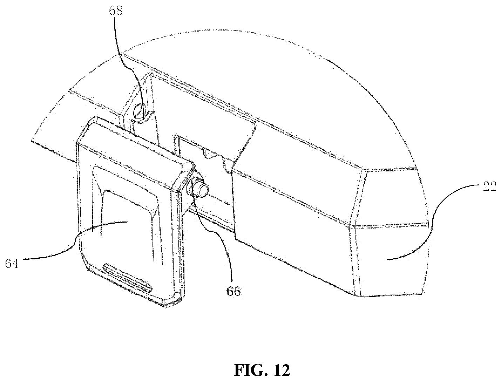

FIG. 12 is a schematic partially enlarged view of the upper cover of the steam cleaning device shown in FIG. 11, in which the a step portion and the matching portion are enlargedly shown. As shown in FIGS. 11 and 12, in some embodiments, two sides of the lock 64 are provided with a step portion 66 respectively, and the upper cover 22 includes a matching portion 68 for matching with the step portion 66.

With this arrangement, it is beneficial to a locking strength.

The step portion 66 and the matching portion 68 may have a step structure with a shape and size corresponding to each other. During transportation, the step portion 66 and the matching portion 68 contact each other to enhance the strength of the lock 64 and secure the locking of the box body 12.

Still referring to FIG. 9, in some embodiments, a bottom of the box body 12 is provided with a universal wheel 70.

With this arrangement, it is beneficial to move the steam cleaning device 10 conveniently and easily.

Although the present disclosure has been disclosed above, the present disclosure is not limited thereto. Any changes and modifications may be made by those skilled in the art without departing from the spirit and scope of the present disclosure, and the scope of the present disclosure should be determined by the appended claims.

## Claims

1. A steam cleaning device (10), comprising:
a box body (12) internally provided with a housing space (14), wherein the housing space (14) comprises a plurality of mutually independent storage portions (16);
a steam generating device (18) arranged inside the box body (12) and outside the housing space (14); and
an accessory (20) capable of being in fluid communication with or connected with the steam generating device (18) and adapted to be correspondingly accommodated in the plurality of mutually independent storage portions (16).

2. The steam cleaning device (10) according to claim 1, wherein the housing space (14) is U-shaped.

3. The steam cleaning device (10) according to claim 1 or 2, wherein the box body (12) comprises an upper cover (22), a lower cover (24) arranged below the upper cover (22) and a middle frame (26) arranged between the upper cover (22) and the lower cover (24), and the housing space (14) is arranged in the middle frame (26).

4. The steam cleaning device (10) according to claim 3, wherein the housing space (14) is arranged at an upper edge of the middle frame (26), the steam generating device (18) is arranged below the middle frame (26), and the housing space (14) partially surrounds the steam generating device (18).

5. The steam cleaning device (10) according to claim 3, wherein the accessory (20) comprises a water tank (28) that is in fluid communication with the steam generating device (18), and the plurality of mutually independent storage portions (16) comprise a first storage portion (30) adapted to accommodate the water tank (28).

6. The steam cleaning device (10) according to claim 5, wherein the water tank (28) comprises a cover (32) made of an elastomer.

7. The steam cleaning device (10) according to claim 6, wherein an inner side of the upper cover (22) is provided with a support rib (34) that abuts against an upper part of the cover (32).

8. The steam cleaning device (10) according to claim 6, wherein the water tank (28) comprises a handle portion (36) arranged outside a vertical line (L) where a center of gravity (G) of the water tank (28) is located.

9. The steam cleaning device (10) according to claim 8, wherein in a horizontal direction, the handle portion (36) is arranged between the center of gravity (G) of the water tank (28) and the cover (32).

10. The steam cleaning device (10) according to claim 5, wherein the water tank (28) is adapted to be removably accommodated in the first storage portion (30).

11. The steam cleaning device (10) according to claim 5, wherein the water tank (28) is adapted to be fixedly accommodated in the first storage portion (30).

12. The steam cleaning device (10) according to claim 5, wherein the housing space (14) comprises a second storage portion (38) in addition to the first storage portion (30), and the second storage portion (38) is L-shaped.

13. The steam cleaning device (10) according to claim 5, wherein a bottom of the first storage portion (30) is provided with a reference surface (40) and an inlet (42), and a height (H1) of the inlet (42) is greater than a height (H2) of the reference surface (40) relative to an outer bottom surface (41) of the first storage portion (30).

14. The steam cleaning device (10) according to claim 3, wherein an inner side of the upper cover (22) is provided with a holding portion (44) made of a soft rubber material, and the plurality of mutually independent storage portions (16) comprise a third storage portion (46) opposite to the holding portion (44).

15. The steam cleaning device (10) according to claim 14, wherein the accessory (20) comprises a steam nozzle (48) and a steam pipe (50), the holding portion (44) is adapted to accommodate the steam nozzle (48), and the third storage portion (46) is adapted to accommodate the steam pipe (50).

16. The steam cleaning device (10) according to claim 15, wherein a front outer side of the lower cover (22) is provided with a connecting port (52) for connecting the steam pipe (50).

17. The steam cleaning device (10) according to claim 1, wherein the accessory (20) comprises a power wire (54), a side bottom of the box body (12) is provided with a wire outlet (56), and the power wire (54) extends from the wire outlet (56) to an outer side of the box body (12).

18. The steam cleaning device (10) according to claim 17, wherein at least one side of the box body (12) is externally provided with a mesh portion (56) for accommodating the power wire (54).

19. The steam cleaning device (10) according to claim 3, further comprising a handle (58) arranged on an outer side of the upper cover (22).

20. The steam cleaning device (10) according to claim 19, wherein an end of the handle (58) is provided with a mounting guide portion (60), and the outer side of the upper cover (22) is provided with a mounting portion (62) for matching with the mounting guide portion (60).

21. The steam cleaning device (10) according to claim 20, wherein the mounting guide portion (60) has a mounting guide angle θ ranging from 30 to 40 degrees.

22. The steam cleaning device (10) according to claim 3, wherein the upper cover (22) is capable of being opened and closed relative to the lower cover (24).

23. The steam cleaning device (10) according to claim 22, wherein a front side of the upper cover (22) is provided with a lock (64) for locking the box body (12).

24. The steam cleaning device (10) according to claim 23, wherein two sides of the lock (64) are provided with a step portion (66) respectively, and the upper cover (22) comprises a matching portion (68) for matching with the step portion (66).

25. The steam cleaning device (10) according to claim 1, wherein a bottom of the box body (12) is provided with a universal wheel (70).
